# FASCICULE DE BREVET EUROPEEN

(11) **EP 3 002 043 B1**
(45) Date de publication et mention de la délivrance du brevet: **26.02.2020**
(21) Numéro de dépôt: 15290237.5
(22) Date de dépôt: 22.09.2015
(51) Int. Cl.: A61Q 17/04, A61Q 19/04, A61K 8/72, A61K 8/9789

(54) **PROCÉDÉ DE PRÉPARATION D'UN EXTRAIT D'OLIVE À TENEUR EN MÉLANINE SUPÉRIEURE À 5%**
VERFAHREN ZUR HERSTELLUNG EINES OLIVENEXTRAKT MIT EINEM MELANIN-GEHALT VON MEHR ALS 5%
A METHOD FOR PREPARING AN OLIVE EXTRACT WITH A MELANIN CONTENT GREATER THAN 5%

(30) Priorité: 02.10.2014 FR 1402222
(43) Date de publication de la demande: 06.04.2016
(73) Titulaire: Biocyte, 06250 Mougins (FR); Monin, Claude, 06530 Cabris (FR)
(72) Inventeur: Monin, Claude, 06530 Cabris (FR); Bruneau, Philippe, 06110 Le Cannet (FR)
(74) Mandataire: Macquet, Christophe

(56) Documents cités:
- EP-A2- 1 421 930
- WO-A1-00/64472
- WO-A1-02/07696
- WO-A1-95/09629
- DE-A1-102004 003 801
- DE-A1-102007 006 792
- US-A- 5 256 403
- US-A- 5 804 168
- US-A1- 2005 089 483
- DATABASE WPI Week 201246 Thomson Scientific, London, GB; AN 2012-E60729 XP002739873, & CN 102 406 582 A (ZENG Q) 11 avril 2012 (2012-04-11)
- DATABASE GNPD [Online] MINTEL; avril 2014 (2014-04), "Self-Tanning Protector Dietary Supplement with Melanin, Tyrosine & Copper", XP055190704, Database accession no. 1985107
- DATABASE GNPD [Online] MINTEL; février 2014 (2014-02), "Sun & Protection Face Cream SPF 50", XP002739874, Database accession no. 1985113
- DATABASE GNPD [Online] MINTEL; juin 2013 (2013-06), "Sun Tan & Protection Body Cream SPF 50", XP002739875, Database accession no. 1985097
- Carole Paufique: "Un, deux, trois... soleil", Le Figaro.fr Madame, 20 juin 2013 (2013-06-20), pages 1-5, XP055190543, Extrait de l'Internet: URL:http://madame.lefigaro.fr/beaute/deux- trois-soleil-200613-391596 [extrait le 2015-05-20]
- DATABASE WPI Week 201020 Thomson Scientific, London, GB; AN 2010-C31527 XP002739876, & CN 101 643 589 A (GANSU JINGYE AGRIC SCI & TECHNOLOGY CO LTD) 10 février 2010 (2010-02-10)

## Description

La présente invention concerne un nouveau procédé d'extraction de la mélanine. Plus particulièrement, l'invention concerne un procédé d'extraction de la mélanine à partir d'une matière première naturelle qui est l'olive noire. L'invention concerne également un produit à base de mélanine susceptible d'être obtenu par un tel procédé. Est décrit également l'utilisation d'un tel produit, notamment pour le bronzage et/ou le brunissement de la peau, et éventuellement pour la protection de la peau contre les rayonnements UV-A et/ou UV-B.

La mélanine fait partie des principaux pigments de couleur situés dans l'épiderme. Tous les individus ne possèdent pas le même taux de mélanine. Des facteurs héréditaires ou hormonaux conditionnent généralement cette concentration. La mélanine est bien connue pour son rôle essentiel en matière de protection de la peau face aux rayonnements ultraviolets (UV) qui sont notamment responsables :
- du photo-vieillissement, c'est-à-dire la modification de l'orientation des fibres d'élastine et de collagène, entraînant un relâchement et une perte de fermeté de la peau et un vieillissement prématuré par l'apparition de rides ;
- des allergies solaires ou photo-allergies (rougeurs, démangeaisons, lucites estivales) ;
- des désordres pigmentaires (masque de grossesse, taches) ; et
- du développement de cancers cutanés.

Généralement, la quantité de mélanine évolue proportionnellement au phototype de la peau. Ainsi, un individu à la peau mate aura plus de pigments de mélanine qu'un individu à la peau claire. La concentration de mélanine fabriquée désigne le phototype d'une personne.

La mélanogénèse est le processus de synthèse et de distribution de la mélanine dans l'épiderme. La mélanogénèse se déroule en plusieurs étapes successives :
(i) les mélanocytes, présents au niveau de la membrane basale de l'épiderme, produisent des organites rugueux appelés mélanosomes ;
(ii) les mélanosomes synthétisent de la mélanine, pigment responsable notamment de la coloration cutanée ;
(iii) les mélanosomes, une fois chargés en mélanines, migrent vers l'extrémité des dendrites du mélanocyte et sont ensuite transférés vers les kératinocytes où ils libèrent la mélanine ; et
(iv) les kératinocytes remontent alors à la surface de la peau qui se pigmente.

Sous l'effet des rayonnements ultraviolets (UV) toutes les étapes de la mélanogénèse sont stimulées.

La mélanine est connue pour protéger la peau des rayonnements ultraviolets (UV). C'est un excellent absorbant de rayonnements ultraviolets (UV). La mélanine protège les organismes des lésions de l'ADN causées par les rayonnements ultraviolets (UV) et du stress environnemental. La mélanine possède donc des applications potentielles en cosmétique, en médecine et particulièrement en dermatologie, pour les lentilles optiques, pour les peintures et les vernis.

Le document intitulé *"*Compton scattering by internal shields based on melanin-containing mushrooms provides protection of gastrointestinal tract from ionizing radiation." (Dadachova et al., Cancer Biother Radiopharm. 2012) suggère également que la mélanine puisse être utilisée dans la protection contre les radiations gamma. Elle pourrait notamment être utilisée contre des actions terroristes utilisant des dispositifs radiologiques et pour protéger les astronautes dans l'espace, ainsi que passagers et équipage à bord des avions de lignes. En effet, à l'altitude de croisière des avions de lignes, soit environ 10 000 m à 12 000 m, le rayonnement cosmique est environ 100 à 300 fois plus intense qu'au niveau de la mer, et il n'existe aucun moyen physique capable d'arrêter les rayonnements cosmiques qui traversent les carlingues des avions.

Le document intitulé *"*Melanin, a promising radioprotector: mechanisms of actions in a mice model" (Kunwar et al., Toxicol Appl Pharmacol. 2012) divulgue également que la mélanine prévient l'apoptose dans les tissus spléniques et réduit le stress oxydatif dans les tissues hépatiques.

Le mécanisme de synthèse de la mélanine est complexe. La mélanine est généralement produite à partir de tyrosine grâce à l'enzyme tyrosinase. La tyrosine est tout d'abord convertie en DOPA, puis en dopaquinone. En l'absence de cystéine, de l'indole-5,6-quinone est formé, donnant l'eumélanine. En présence de cystéine, de la cystéinyldopa est formée, donnant de la phéomélanine. Ces deux pigments polymérisent à un degré variable selon leur nature.

Compte tenu de son intérêt, des procédés de synthèse de mélanine ont été développés. Ainsi, le document US5218079 décrit la synthèse de mélanine à partir de dopachrome et de 5,6-dihydroxyindole. Toutefois, une telle synthèse *in vitro* est généralement très coûteuse par rapport à une production de mélanine à partir de produits naturels. En outre, de tels procédés de synthèse de mélanine synthétique nécessitent l'utilisation de produits chimiques pouvant être nocifs pour l'environnement.

Le document EP1052290 décrit un procédé de synthèse de biomélanine à partir de la fermentation du champignon *Podospora anserina* de la famille des *Sondariaceae.* Toutefois, un tel procédé est relativement fastidieux et long à mettre en place pour une production de mélanine à grande échelle.

Il existe donc un besoin de développer un procédé d'extraction de mélanine qui soit facile à mettre en œuvre, rapide, et peu coûteux. La mélanine actuellement commercialisée, d'origine naturelle, provient notamment d'animaux, par exemple l'encre de seiche, ou de végétaux par exemple la coque de châtaigne. Toutefois, les produits actuels utilisés ne permettent pas d'avoir un rendement suffisant permettant de produire de grandes quantités de mélanine à un coût raisonnable. Il demeure donc un besoin de disposer d'un procédé de fabrication de mélanine à partir d'une matière première qui soit commune, abondante et donc disponible en grande quantité.

La solution au problème posé a pour premier objet un procédé de préparation d'un extrait d'olive à teneur en mélanine supérieure à 5% en poids du poids total de l'extrait comprenant les étapes suivantes de :
- mélange d'une matière première issue de l'olive choisie parmi des grignons d'olives noires et/ou des margines d'olives noires avec une solution aqueuse alcaline, ladite matière première a une concentration en lipides inférieure ou égale à 10% en poids du poids total de ladite matière;
- macération du mélange obtenu ;
- filtration du macérât ;
- mélange d'un composé acide au filtrat obtenu ;
- filtration du mélange afin d'obtenir un gâteau humide ;
- séchage du gâteau humide ; et
- récupération d'un extrait solide comprenant une teneur en mélanine supérieure à 5% en poids du poids total de l'extrait.

De façon surprenante, le Demandeur a mis en évidence qu'il était possible de mettre au point un procédé d'extraction de mélanine à partir des sous-produits du processus d'extraction de l'huile d'olive.

L'invention a pour deuxième objet un produit à base de mélanine susceptible d'être obtenu par le procédé selon l'invention.

L'invention sera mieux comprise à la lecture de la description non limitative qui suit, rédigée au regard des dessins annexés, dans lesquels les figures la à 1f représentent des coupes histologiques permettant de démontrer que les produits selon l'invention augmentent la teneur en mélanine sur la peau. La visualisation de la mélanine dans les tissus étudiés, reprise aux figures la à 1f est faite suivant la technique de Masson, variante de Fontana.
- la figure la correspond au témoin négatif, sans application ;

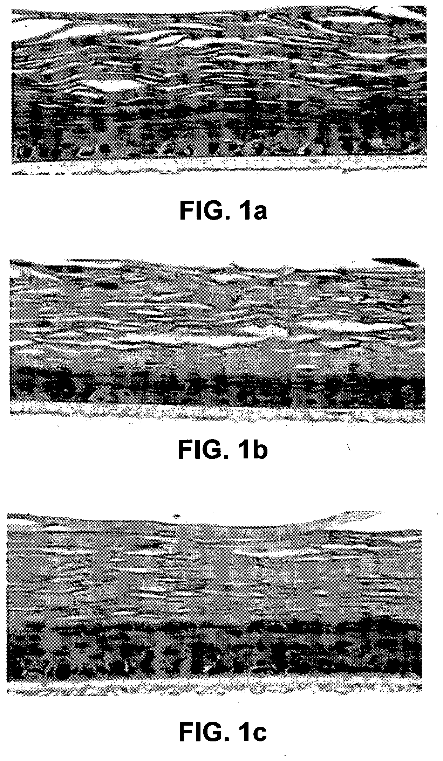

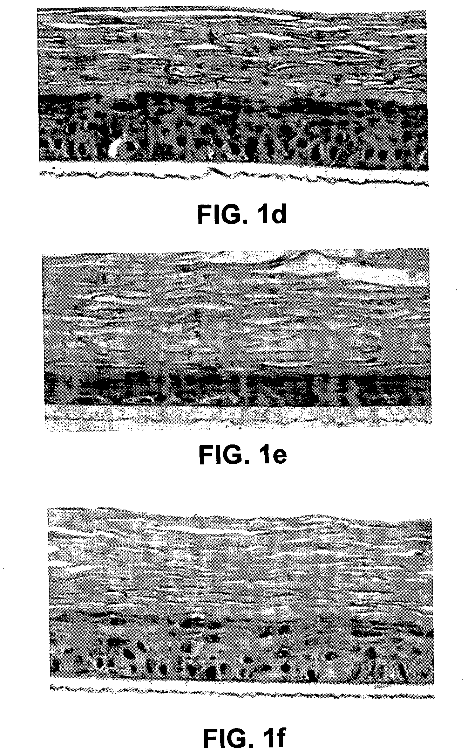

- la figure 1b correspond au tissu sur lequel de la mélanine à 1% est appliquée en topique ;
- la figure 1c correspond au tissu sur lequel de l'alpha-MSH est appliquée en systémique ;
- la figure 1d correspond au témoin négatif, sans application, mais soumis à un rayonnement UVB ;
- la figure le correspond au tissu sur lequel de la mélanine à 1% est appliquée en topique, et qui est soumis à un rayonnement UVB ;
- la figure 1f correspond au tissu sur lequel de l'alpha-MSH est appliquée en systémique, et qui est soumis à un rayonnement UVB.

Le Demandeur a mis au point un procédé d'extraction d'un extrait d'olive à teneur en mélanine supérieure à 5% en poids du poids total de l'extrait à partir d'une matière première issue de l'olive.

Selon l'invention, le terme olive désigne le fruit de l'arbre de la famille des oléacées du genre *Osmanthus* et/ou *Olea.* De préférence, l'olive est le fruit des espèces *Osmanthus fragrans, Olea fragrans* ou *Olea europaea.* De préférence encore, l'olive est le fruit de l'olivier *Olea europaea.*

Selon l'invention, la matière première utilisée est choisie parmi l'olive noire écrasée après transformation également appelée grignons, les margines (appelées également eaux de végétation) ou les « grignons pâteux » appelés également « marginions » qui sont des grignons à teneur en eau plus élevée que les grignons (teneur en eau supérieure ou égale à 15%). La matière première issue de l'olive utilisée a une concentration en lipides inférieure ou égale à 10% en poids du poids total.

Ainsi, selon l'invention la matière première issue de l'olive est choisie parmi des grignons d'olives noires et/ou des margines d'olives noires. Cette matière première est, de façon avantageuse, particulièrement disponible en Europe du Sud et en Afrique du Nord, ce qui facilite sa production dans ces pays.

Il est connu que l'huile d'olive peut notamment être extraite à la suite de l'ensemble des étapes suivantes :
(i) étape de broyage permettant de briser les parois des cellules et d'en faire sortir les sucs. Le produit de cette phase est généralement appelé pâte ;
(ii) étape d'extraction du moût d'huile qui permet de séparer la phase liquide, l'émulsion eau-huile (moût d'huile ou jus d'olives composé de margines et d'huile), de la phase solide (grignons) ; et
(iii) étape de séparation de l'huile et de l'eau qui permet de séparer les deux composants du moût d'huile. Au cours de ce processus on sépare les deux phases liquides non miscibles et une grande partie des dépôts. La phase aqueuse résiduelle est appelée margines tandis que la phase huileuse est appelée huile d'olives.

Les sous-produits du processus d'extraction de l'huile d'olive sont :
- les grignons, composé des peaux, des résidus de la pulpe et des fragments des noyaux ; et
- les margines, résidus liquide résultant de l'extraction d'huile.

L'avantage conféré par l'utilisation de sous-produits du processus d'extraction de l'huile d'olive est avant tout environnemental. L'utilisation de tels sous-produits diminue la charge polluante des producteurs d'huile d'olive en limitant le traitement et/ou les rejets de ces sous-produits. Un autre avantage est que cette matière première issue du processus d'extraction de l'huile d'olive est moins chère que l'olive entière. Enfin, un dernier avantage conféré par l'utilisation de sous-produits exempts ou partiellement exempts d'huile d'olive est qu'il va permettre d'accélérer le procédé de préparation selon l'invention. En effet, les filtrations sont plus lentes lorsque la matière première comprend de l'huile d'olive. La présence d'huile d'olive peut en outre nécessiter l'utilisation d'adjuvants de filtration tels que les silices.
Ainsi, de façon particulièrement avantageuse, la matière première utilisée est faite de grignons, sous-produits du processus d'extraction de l'huile d'olive.

Le procédé selon l'invention comprend une première étape de mélange d'une matière première issue de l'olive telle que définie ci-dessus avec une solution aqueuse alcaline. La solution aqueuse alcaline est avantageusement obtenue par dissolution de potasse ou de soude caustique ou tout autre composé alcalin dans l'eau. Le pH du milieu alcalin doit être au-dessus de 8, avec un intervalle préféré compris entre 8 et 12.

La solution aqueuse alcaline est préférentiellement une solution aqueuse de soude (NaOH) ou de potasse (KOH). De préférence, la solution aqueuse alcaline comprend entre 1% et 15% de soude (NaOH), en poids du poids total de la solution. De préférence encore, la solution aqueuse alcaline comprend entre 2% et 5% de soude (NaOH), en poids du poids total de la solution.

Le procédé selon l'invention comprend une deuxième étape de macération du mélange obtenu. De préférence l'étape de macération est réalisée pendant 1 à 3 heures à une température comprise entre 20°C et 100°C. De préférence encore, l'étape de de macération est réalisée pendant 2 heures à une température comprise entre 50°C et 80°C.

Le procédé selon l'invention comprend également des étapes de filtration du macérât et de mélange d'un composé acide au filtrat obtenu. L'étape de filtration peut par exemple être réalisée sur filtre Büchner sous vide pendant 1 à 7 heures. De préférence, le composé acide est choisi parmi l'acide citrique (acide 2-hydroxypropane-1,2,3-tricarboxylique), l'acide acétique (acide éthanoïque), l'acide sulfurique (sulfate d'hydrogène) ou l'acide chlorhydrique (HCl) ou tout autre composé acide permettant au milieu d'avoir un pH inférieur à 7, de préférence compris entre 7 et 1.

De préférence, l'étape de séchage du gâteau humide du procédé selon l'invention est réalisée au moyen de techniques connues telles que notamment la lyophilisation, le séchage par pulvérisation, le séchage au four, le four ventilé ou le four sous vide.

Enfin, le procédé selon l'invention comprend une étape de récupération d'un extrait solide comprenant une teneur en mélanine supérieure à 5% en poids du poids total de l'extrait. De préférence, la teneur en mélanine est comprise entre 5% et 50% en poids du poids total de l'extrait. De préférence encore, la teneur en mélanine est comprise entre 10% et 30% en poids du poids total de l'extrait.

L'invention concerne également un produit à base de mélanine, qui est un extrait d'olive à teneur en mélanine supérieure à 5%, susceptible d'être obtenu par le procédé selon l'invention.

Le produit à base de mélanine susceptible d'être obtenu par le procédé selon l'invention est formulé dans une composition cosmétique topique et/ou orale. Ladite composition cosmétique est utilisée préférentiellement pour le bronzage et/ou le brunissement de la peau. De préférence encore, la composition est également adaptée à la protection de la peau contre les rayonnements UV-A et/ou UV-B.

A notre époque, il est important, pour des questions esthétiques et sociales, d'avoir une peau bronzée ou hâlée. Une peau bronzée ou hâlée est synonyme de bonne mine et de bonne santé. Cependant, le bronzage n'est pas toujours souhaitable dans la mesure où il nécessite des expositions prolongées aux rayonnements solaires. Les rayonnements solaires sont connus pour avoir des effets bénéfiques mais surtout des effets néfastes chez l'Homme. Les rayonnements solaires possèdent notamment une action antirachitique, en aidant à la synthèse de vitamine D, améliore certaines dermatoses, présente une action antidépressive, et permet le brunissement de la peau. Toutefois, une surexposition aux rayonnements solaires est susceptible d'induire une altération de la peau. Les rayonnements solaires comprennent des rayonnements ultraviolets (UV) qui sont très puissants. Parmi les ultraviolets, seuls les UV-A et les UV-B atteignent la terre et présentent des effets sur la peau. Les UV-C sont, quant à eux, arrêtés par la couche d'ozone. Les rayons UV-A, de longueurs d'onde comprises entre 320 et 400 nm, provoquent le brunissement de la peau. Ils sont présents toute l'année et même par temps nuageux. Ils représentent 95% des UV qui touchent la surface de la terre. Contrairement aux UV-B, ils sont indolores et peuvent pénétrer la peau très profondément, jusqu'aux cellules du derme. Les UV-A provoquent en particulier :
- le photo-vieillissement, c'est-à-dire la modification de l'orientation des fibres d'élastine et de collagène, entraînant un relâchement et une perte de fermeté de la peau et un vieillissement prématuré par l'apparition de rides ;
- des allergies solaires ou photo-allergies (rougeurs, démangeaisons, lucites estivales) ;
- des désordres pigmentaires (masque de grossesse, taches) ; et
- le développement de cancers cutanés.

Ainsi, lors d'une exposition aux rayons naturels ou artificiels, il est important de filtrer le rayonnement UV-A. Les rayons UV-B, de longueurs d'onde comprises entre 280 et 320 nm sont très énergétiques. Les rayons UV-B sont responsables du bronzage mais aussi des brûlures (coups de soleil) ainsi que de réactions allergiques et de cancers cutanés. Compte tenu de ce qui précède, il apparait donc essentiel de protéger la peau à la fois des UV-A et des UV-B.

Les compositions solaires actuelles comprennent généralement plusieurs filtres solaires. De telles compositions solaires sont notamment décrites dans le document FR2975590 ou dans le document FR 2983718.

Toutefois, la présence de tels filtres solaires présentant un indice de protection élevé, c'est-à-dire d'au moins 25, préférentiellement d'au moins 30 est généralement incompatible avec un bronzage rapide de la peau.

Aussi, le Demandeur a développé des compositions cosmétiques topiques et/ou orales comprenant un extrait d'olive à teneur en mélanine supérieure à 5% obtenu selon le procédé de l'invention. De telles compositions permettent d'une part de favoriser le bronzage, mais protègent également la peau face aux rayonnements ultraviolets (UV). Ceci ressort notamment des exemples 2 et 3 ci-dessous.

Les compositions topiques peuvent se présenter par exemple sous la forme d'une solution huileuse, d'une lotion, d'une émulsion obtenue par dispersion d'une phase grasse dans une phase aqueuse (H/E) ou inversement (E/H), ou d'une émulsion triple (E/H/E ou H/E/H), d'une crème, d'un gel, d'un gel-spray, d'un spray, d'une pommade ou d'une mousse.

De préférence, la composition topique est une crème.

Selon un mode de réalisation préféré, la composition topique comprend au moins un filtre UV. A titre d'exemple non limitatif de filtre UV utilisable, on peut citer :
- les dérivés de la 1,3,5-triazine-2,4,6-triamine, tels que le Diethylamino Hydroxybenzoyl Hexyl Benzoate (Uvinul™ A+), l'Ethyl Hexyl Triazone (Uvinul™ T 150), la Bis-ethyl Hexyloxyphenol Methoxyphenyl Triazine (Tinosorb™ S), l'Octocrylène (Eusolex® OCR), le Dimethicodiethylbenzalmalonate (Parsol® SLX), la Di-ethylhexyl-butamido Triazone (Uvasorb® HEB) ;
- les esters d'acide cinnamique, tels que le p-methoxycinnamate d'isoamyle ou le p-méthoxycinnamate de 2-éthylhexyle (INCI : Ethyl Hexyl Methoxycinnamate), notamment commercialisé sous le nom d'Eusolex® 9020) ;
- les dérivés du phényl benzimidazole, tels que le Phenylbenzimidazole Sulfonix Acid, (Eusolex® 232) ;
- les dérivés de la benzophénone, tels que la Benzophenone-3 (Tinosorb™ B3), la Benzophenone-4 (Uvinul™ MS 40) ou la Benzophenone-5 ; et
- les dérivés de l'acide p-aminobenzoïque, tels que le dérivé éthoxylé PEG 25 - PABA (Uvinul™ P25), le Methylene Bis-benzotriazolyl Tetramethylbutylphenol (Tinosorb™ M).

De préférence, la composition comprend au moins un, deux ou trois filtres UV choisis parmi le Bis-Ethylhexyloxyphenol Methoxyphenyl Triazine (BEMT) (Tinosorb™ S), l'ethylhexyl Methoxycinnamate ou le diethylamino Hydroxybenzoyl Hexyl Benzoate (Uvinul™ A+).

Selon un mode de réalisation avantageux la composition topique comprend un, deux ou trois filtres UV tels que ci-dessus, à des concentrations permettant l'obtention d'un indice de protection élevé, c'est-à-dire d'au moins 25, préférentiellement d'au moins 30. Plus préférentiellement encore, l'indice de protection de la composition topique est de 50.

Les compositions orales peuvent se présenter par exemple sous la forme d'une poudre, de cachet, de gélule, de pilule, de granule, de tablette, de pastilles, de comprimés ou de sirop.

Les compositions topiques ou orales peuvent comprendre en outre tout additif usuellement utilisé dans le domaine pharmaceutique, dermatologique, ou cosmétique compatible avec la mélanine.

Selon un mode de réalisation particulier, la demande décrit un produit de combinaison associant d'une part une composition topique telle que définie ci-dessus et d'autre part une composition orale telle que définie ci-dessus.

Un tel produit de combinaison peut être avantageusement administré de manière simultanée, séparée ou étalée dans le temps, pour le bronzage et/ou le brunissement de la peau et/ou pour la protection de la peau contre les rayonnements UV-A et/ou UV-B.

Enfin, la demande décrit également l'utilisation d'un produit ou d'une composition pour le bronzage et/ou le brunissement de la peau et/ou pour son utilisation dans la protection de la peau contre les rayonnements UV-A et/ou UV-B. De préférence, le produit ou la composition est utilisé à la fois pour le bronzage, le brunissement de la peau et pour la protection de la peau contre les rayonnements UV-A et/ou UV-B.

Plus préférentiellement, les compositions sont particulièrement efficaces pour protéger la peau humaine contre les effets nocifs des rayonnements UV-A et/ou UV-B qui comprennent mais ne sont pas limités au photo-vieillissement, aux brûlures, aux allergies solaires ou photo-allergies, aux désordres pigmentaires et au développement de cancers cutanés.

La présente invention va maintenant être illustrée au moyen des exemples suivants. Dans ces exemples, les quantités sont exprimées en pourcentage pondéral.

### Exemple 1 : Procédé d'extraction selon l'invention :

Pour la fabrication d'un extrait d'olive à teneur en mélanine supérieure à 5% en poids du poids total de l'extrait, le Demandeur a optimisé le procédé de préparation suivant :
A partir d'environ 150g de pâte d'olives noires (tapenade) ou de grignons d'olives noires, il convient de procéder à une extraction avec 300 ml de solution aqueuse de soude à 3% (soit 9g de NaOH).

Le mélange est agité pendant environ 2 heures à une température d'environ 65°C.

Puis, le mélange est filtré sur filtre Büchner sous vide pendant une durée d'environ 4 heures.

Le gâteau obtenu est ensuite lavé deux fois avec 2x10 ml d'eau. Le gâteau alcalin est écarté.

Dans le filtrat, de l'acide chlorhydrique est ajouté sous agitation jusqu'à obtenir un pH de 3. La quantité d'acide chlorhydrique ajoutée est d'environ 17 ml. La mélanine précipite après agitation pendant environ 2 heures à température ambiante (environ 20°C) et est laissé à reposer pendant une nuit.

Puis le mélange est filtré sur filtre Büchner sous vide pendant environ 2 heures. Le gâteau obtenu est lavé deux fois avec 2x10 ml d'eau.

Le gâteau essoré humide est alors récupéré puis mis à sécher à l'étuve à une température d'environ 90°C jusqu'à obtention d'un poids constant, soit pendant environ 4 heures. Le résidu est passé au mortier et permet l'obtention d'un produit noir sous forme de poudre.

Le rendement de l'opération est d'environ 3% sur la matière première.

### Exemple 2 : Mise en évidence de l'activité pro-pigmentante d'une composition comprenant de la mélanine selon l'invention sur des explants de peau humaine maintenus en survie :

Le Demandeur a réalisé une étude dont le but est de montrer l'activité pro-pigmentante d'une composition comprenant de la mélanine selon l'invention sur des explants de peau humaine maintenus en survie.

Cette activité a été évaluée par l'observation de la morphologie générale, par la visualisation de la mélanine et par des mesures au chromamètre.

### I. Mode opératoire :

### a. préparation des échantillons :

Un échantillon de poudre de grignons d'olives comprenant environ 15% en poids de mélanine a été mélangé à de l'ammoniaque afin d'obtenir une solution à 5mg/ml de poudre.

### b. préparation des explants :

Quinze explants, d'un diamètre d'environ 10mm, ont été préparés à partir d'une plastie de bras d'une femme âgée de 80 ans. Les explants ont été répartis en cinq lots de trois explants chacun comme cela est détaillé dans le tableau 1 ci-dessous :

**Tableau 1 : répartition des quinze explants :**

| Lot | Produit | Nbre d'explants |
|---|---|---|
| T0 | | 3 |
| T | Témoin non traité | 3 |
| D | Application topique | 3 |
| M | Incorporation dans milieu de survie | 3 |
| S | Application topique + Incorporation dans milieu de survie | 3 |

### c. application du produit :

La solution à 5 mg/ml a été appliquée par voie topique a raison de 2,5 µl par explant pour les lots D et S aux jours J0, J1, J2, J4, J6 et J8. La solution a été incorporée dans le milieu de survie a raison de 2 µl pour les lots M et S aux jours J0, J1 , J2, J4, J6 et J8.

### d. prélèvement pour l'histologie :

A T0, les trois explants du lot T0 ont été prélevés. Chaque explant a été coupé en 2 moitiés : 1 moitié a été fixée dans du formol tamponné, l'autre a été congelée à -80°C.

A J11, trois explants de chaque lot ont été prélevés et traités de la même manière.

### e. Histologie

Après 24 heures de fixation dans le formol tamponné, les prélèvements ont été déshydratés et imprégnés en paraffine à l'aide d'un automate de déshydratation Leica™ 1020. Ils ont été mis en bloc à l'aide d'une station d'enrobage Leica™ EG 1160.

Des coupes de 5µm ont été réalisées à l'aide d'un microtome type Minot, Leica RM 2125 et collées sur des lames de verre histologiques silanisées superfrost.

Les observations microscopiques ont été réalisées en microscopie optique, à l'aide d'un microscope Leica Orthoplan couplé à une caméra tri CCD Sony™ DXC 390P et le logiciel Leica™ IM1000.

### i. Morphologie générale

L'observation de la morphologie générale a été effectuée sur coupes paraffine colorées au trichrome de Masson, variante de Goldner.

### ii. Visualisation de la mélanine

La visualisation de la mélanine a été réalisée sur coupes paraffine par imprégnation à l'argent suivant la technique de Masson, variante de Fontana.

### II. Résultats de morphologie générale et de visualisation de la mélanine :

### a. lot témoin à J0 :

Le stratum corneum est assez épais, très légèrement feuilleté, modérément kératinisé en surface et très nettement kératinisé à sa base avec une parakératose modérée. L'épiderme présente cinq à six assises cellulaires avec une bonne morphologie. Le relief de la jonction dermo-épidermique est faible. Le derme papillaire présente un collagène avec des fibres épaisses formant un réseau dense. Il est bien cellularisé.

La mélanine est nette et assez régulière dans les kératinocytes de la couche basale. Elle est assez régulièrement répartie dans le cytoplasme. Elle est très peu présente dans les couches suprabasales. Les mélanocytes présentent une bonne morphologie. Ils sont très modérément chargés en mélanine et sont modérément dendritiques.

### b. lot non traité à J11 (T J11) :

Le stratum corneum est épais, modérément feuilleté, modérément kératinisé en surface et très nettement kératinisé à sa base avec une nette parakératose. L'épiderme présente cinq à six assises cellulaires avec une bonne morphologie. Le relief de la jonction dermo-épidermique est très faible. Le derme papillaire présente un collagène avec des fibres épaisses formant un réseau dense. Il est bien cellularisé.

La mélanine est assez nette et très irrégulière dans les kératinocytes de la couche basale. Elle est assez régulièrement répartie dans le cytoplasme.

Elle est peu présente dans les couches suprabasales.

Les mélanocytes présentent une bonne morphologie. Ils sont légèrement chargés en mélanine et sont peu dendritiques.

### c. lot traité avec le produit en topique à J11 (DJ11) :

Le stratum corneum est épais, assez bien feuilleté, légèrement kératinisé en surface et très nettement à sa base avec une nette parakératose. L'épiderme présente cinq à six assises cellulaires avec une bonne morphologie. Le relief de la jonction dermo-épidermique est très faible. Le derme papillaire présente un collagène avec des fibres épaisses formant un réseau assez dense. Il est bien cellularisé.

La mélanine est très nette et assez régulière dans les kératinocytes de la couche basale. Elle est plus ou moins rassemblée au-dessus du noyau. Elle est modérément présente dans les couches suprabasales. Les mélanocytes présentent une bonne morphologie. Ils sont légèrement chargés en mélanine et sont très modérément dendritiques.

### d. lot traité avec le produit incorporé dans le milieu à J11 (MJ11) :

Le stratum corneum est très épais, modérément feuilleté, modérément kératinisé en surface et très nettement à sa base avec une parakératose modérée. L'épiderme présente trois à quatre assises cellulaires avec une morphologie modérément altérée. Dans les couches suprabasales, de nombreuses cellules légèrement œdématiées. La spongiose est faible en basale. Le relief de la jonction dermo-épidermique est très faible. Le derme papillaire présente un collagène avec des fibres épaisses formant un réseau plus ou moins dense. Il est bien cellularisé.

La mélanine est nette et très irrégulière dans les kératinocytes de la couche basale. Elle est plus ou moins régulièrement répartie dans le cytoplasme. Elle est nettement et irrégulièrement présente dans les couches suprabasales. Les mélanocytes présentent une bonne morphologie. Ils sont assez nettement charges en mélanine et sont modérément dendritiques.

### e. lot traité avec le produit en topique + produit incorporé dans le milieu à J11 (SJ11) :

Le stratum corneum est très épais, légèrement feuilleté, modérément kératinisé en surface et très nettement à sa base avec une très nette parakératose.

L'épiderme présente dix à onze assises cellulaires avec une morphologie modifiée. Dans les couches suprabasales, toutes les cellules sont œdématiées avec noyau pycnotique et œdème périnucléaire.

La spongiose est très nette avec une légère acantholyse en basale. Le relief de la jonction dermo-épidermique est faible. Les mélanocytes présentent un noyau plus ou moins pycnotique. Le derme papillaire présente un collagène avec des fibres épaisses formant un réseau assez dense. Il est bien cellularisé.

La mélanine est très nette et très irrégulière dans les kératinocytes de la couche basale. Elle est plus ou moins régulièrement répartie dans le cytoplasme. Elle est nettement présente dans les couches suprabasales.

Les mélanocytes présentent un noyau plus ou moins pycnotique. Ils sont très légèrement chargés en mélanine et sont peu dendritiques.

### II. Résultats de chromamétrie :

La chromamétrie est une mesure objective de la couleur telle qu'elle est perçue par l'œil humain, et cela dans le cadre d'une définition normée de la couleur.

### a. principe de mesure :

Les mesures de couleur ont été réalisées avec un spectrophotomètre CM2600d avec les réglages suivants :
- Champ de vision standard de 2° comme le définit la CIE en 1931 ;
- Illuminant D65 correspondant à la lumière du jour ;
- Zone de mesure de 3 mm ;
- Espaces de couleur utilisés L*a*b* et L*C*h*
- Méthode SCI (spéculaire inclus), permettant de s'affranchir de l'état de surface.

### b. Etude de la clarté :

La mélanine absorbant toutes les longueurs d'onde du spectre visible assombrit globalement les couleurs de la peau d'où l'utilisation du paramètre L* permettant de mesurer le degré de pigmentation de la peau. Dans l'espace couleur L* a* b*, le paramètre L* correspond à la clarté. Donc si les valeurs de ce paramètre diminuent, il est permis de conclure que le produit a un pouvoir pro-pigmentant.

### c. Etude de l'angle ITA (pour angle typologique individuel) :

- Calcul de I'ITA: arctang[(L*-50)/b*)]

Ainsi, plus l'indice ITA augmente, moins la couleur de la peau est foncée.

Les résultats des différentes valeurs mesurées sont repris dans le tableau 2 ci-dessous :

**Tableau 2 :**

| | L* | a* | b* | ITA | ΔL* | Δ a* | Δ b* | Δ ITA | Clarté | % var. | Moy. |
|---|---|---|---|---|---|---|---|---|---|---|---|
| T1J0 | 58,17 | 4,31 | 17,30 | 25,28 | - | - | - | - | - | - | 2% |
| T1J11 | 59,69 | 3,52 | 12,53 | 37,72 | 1,52 | -0,78 | -4,78 | 12,44 | 1,52 PC | 3% | |
| T2J0 | 57,94 | 4,68 | 18,79 | 22,91 | - | - | - | - | - | - | |
| T2J11 | 58,66 | 3,84 | 13,42 | 32,83 | 0,72 | -0,84 | -5,37 | 9,93 | 0,72 PC | 1% | |
| T3J0 | 60,90 | 2,99 | 18,09 | 31,07 | - | - | - | - | - | - | |
| T3J11 | 62,46 | 3,41 | 13,18 | 43,39 | 1,57 | 0,42 | -4,91 | 12,32 | 1,57 PC | 3% | |
| D1J0 | 59,80 | 4,10 | 18,47 | 27,95 | - | - | - | - | - | - | -8% |
| D1J11 | 55,35 | 4,11 | 15,68 | 18,84 | -4,45 | 0,01 | -2,79 | -9,11 | 4,45 PF | -7% | |
| D2J0 | 58,98 | 4,24 | 16,52 | 28,53 | - | - | - | - | - | - | |
| D2J11 | 54,27 | 5,05 | 17,69 | 13,57 | -4,71 | 0,81 | 1,17 | -14,96 | 4,71 PF | -8% | |
| D3J0 | 59,30 | 3,42 | 16,70 | 29,11 | - | - | - | - | - | - | |
| D3J11 | 54,36 | 3,40 | 13,45 | 17,96 | -4,93 | -0,01 | -3,25 | -11,15 | 4,93 PF | -8% | |
| M1J0 | 58,59 | 4,71 | 18,52 | 24,88 | - | - | - | - | - | - | -3% |
| M1J11 | 56,49 | 3,39 | 13,04 | 26,46 | -2,1 | -1,32 | -5,48 | 1,58 | 2,10 PF | -4% | |
| M2J0 | 55,29 | 5,06 | 16,95 | 17,33 | - | - | - | - | - | - | |
| M2J11 | 54,11 | 3,82 | 11,30 | 19,99 | -1,18 | -1,24 | -5,65 | 2,65 | 1,18 PF | -2% | |
| M3J0 | 59,81 | 4,81 | 17,79 | 28,87 | - | - | - | - | - | - | |
| M3J11 | 58,10 | 2,90 | 11,28 | 35,68 | -1,71 | -1,91 | -6,51 | 6,81 | 1,71 PF | -3% | |
| S1J0 | 58,17 | 3,93 | 17,54 | 24,98 | - | - | - | - | - | - | -10% |
| S1J11 | 51,48 | 4,35 | 13,59 | 6,22 | -6,69 | 0,42 | -3,95 | -18,76 | 6,69 PF | -12% | |
| S2J0 | 59,50 | 4,28 | 18,14 | 27,64 | - | - | - | - | - | - | |
| S2J11 | 52,45 | 4,60 | 13,32 | 10,42 | -7,05 | 0,32 | -4,82 | 17,22 | 7,05 PF | -12% | |
| S3J0 | 56,99 | 2,68 | 16,01 | 23,59 | - | - | - | - | - | - | |
| S3J11 | 53,91 | 3,40 | 8,40 | 24,96 | -3,08 | 0,72 | -7,62 | 1,37 | 3,08 PF | -5% | |

avec :
- « % var » correspondant au pourcentage de variation ;
- « Moy. » signifiant à la moyenne ;
- « Δ » signifiant delta ;
- « PC » signifiant « plus clair » ;
- « PF » signifiant « plus foncé ».

Comme il ressort du tableau 2, le Demandeur a pu constater qu'après onze jours de survie :
- le lot témoin s'éclaircit de 2% ;
- le lot D présente une activité pro-pigmentante de +8% ;
- le lot M présente une activité pro-pigmentante de +3% ;
- le lot S présente une activité pro-pigmentante de +10%.

Ainsi, il apparait que l'incorporation du produit en topique et/ou dans le milieu de survie permet l'obtention d'une activité pro-pigmentante.

Le produit testé aux concentrations indiquées préalablement induit après onze jours de survie une nette augmentation de la mélanine dans l'épiderme.

L'application en topique induit une activité pro-pigmentante presque exclusivement au niveau de la couche basale.

L'incorporation dans le milieu de survie induit une activité pro-pigmentante dans la couche basale et dans les couches supra basales.

Enfin, le produit appliqué en combinaison (en topique et dans le milieu) induit la plus nette activité pro-pigmentante dans la couche basale et dans les couches suprabasales.

Ces observations sont confirmées par les mesures au chromamètre résumées ci-dessous :
- Application topique : activité pro-pigmentante de +8% ;
- Application dans le milieu : activité pro-pigmentante de +3% ;
- Combinaison : activité pro-pigmentante de +10%.

### Exemple 3 : tests de l'effet bronzant d'une composition comprenant de la mélanine sur de l'épiderme :

Le Demandeur a réalisé une étude dont le but est de montrer l'activité pro-pigmentante de compositions comprenant de la mélanine sur de l'épiderme pigmenté reconstruit.

Le modèle d'épiderme pigmenté est reconstruit sur des plaques de 0,5 cm² comprenant un filtre polycarbonate, en utilisant un procédé de reconstruction à l'interface air-liquide. Ce procédé permet une reconstruction tissulaire multicouche comparable au tissu humain *in vivo.*

L'objectif est d'appliquer localement et de façon répétée un concentré de mélanine pendant une durée de six jours sur un épiderme pigmenté reconstruit, puis d'exposer les tissus à une irradiation UV-B.

L'effet sur ces tissus est observé par une étude histologique, par un test de viabilité par dosage MTT (pour 3-[4,5-dimethyl-thiazol-2-yl]-2,5-diphenyl tetrazolium bromide) et par un dosage de la mélanine.

Un témoin positif par application systémique d'alpha MSH (pour *alpha melanocyte stimulating hormone*) et un témoin négatif (tissus non traités) sont utilisés.

Pour chaque condition testée, cinq épidermes sont traités de manière identique. Un pour l'étude histologique, un pour la mesure de la viabilité cellulaire et trois pour le dosage de la mélanine (un pour la mesure de la pigmentation et deux pour l'extraction de la mélanine.

### I. Description des tests:

### a. Application de la mélanine sur le tissu reconstruit :

A partir du onzième jour (J11), un concentré de mélanine à 1% est appliqué topiquement et de façon répétée sur dix tissus reconstruits selon les conditions décrites dans le tableau 3 ci-dessous.

**Tableau 3 :**

| | J11 | J12 | J13 | J14 | J17 | J18 | J19 |
|---|---|---|---|---|---|---|---|
| Condition 1 : Application topique de l'extrait de mélanine | 1^{ère} application sur 5 tissus | 2^{ème} appli. sur 5 tissus | 3^{ème} appli. sur 5 tissus | 4^{ème} appli. sur 5 tissus | 5^{ème} appli. sur 5 tissus | 6^{ème} appli. sur 5 tissus | Viabilité : 1 tissu |
| | | | | | | | Histologie : 1 tissu |
| | | | | | | | Mélanine : 3 tissus |
| | 1^{ère} application sur 5 tissus + Exposition aux UVB | 2^{ème} appli. sur 5 tissus + Expo. aux UVB | 3^{ème} appli. sur 5 tissus + Expo. aux UVB | 4^{ème} appli. sur 5 tissus + Expo. aux UVB | 5^{ème} appli. sur 5 tissus + Expo. aux UVB | 6^{ème} appli. sur 5 tissus + Expo. aux UVB | Viabilité : 1 tissu |
| | | | | | | | Histologie : 1 tissu |
| | | | | | | | Mélanine : 3 tissus |
| Condition 2 : Application systémique d'alpha MSH | 1^{ère} application sur 5 tissus | 2^{ème} appli. sur 5 tissus | 3^{ème} appli. sur 5 tissus | 4^{ème} appli. sur 5 tissus | 5^{ème} appli. sur 5 tissus | 6^{ème} appli. sur 5 tissus | Viabilité : 1 tissu |
| | | | | | | | Histologie : 1 tissu |
| | | | | | | | Mélanine : 3 tissus |
| | 1^{ère} application sur 5 tissus + Exposition aux UVB | 2^{ème} appli. sur 5 tissus + Expo. aux UVB | 3^{ème} appli. sur 5 tissus + Expo. aux UVB | 4^{ème} appli. sur 5 tissus + Expo. aux UVB | 5^{ème} appli. sur 5 tissus + Expo. aux UVB | 6^{ème} appli. sur 5 tissus + Expo. aux UVB | Viabilité : 1 tissu |
| | | | | | | | Histologie : 1 tissu |
| | | | | | | | Mélanine : 3 tissus |
| Condition 3 : Témoin négatif, pas d'application. | - | - | - | - | - | - | Viabilité : 1 tissu |
| | | | | | | | Histologie : 1 tissu |
| | | | | | | | Mélanine : 3 tissus |
| | Exposition aux UVB sur 5 tissus | Expo. aux UVB | Expo. aux UVB | Expo. aux UVB | Expo. aux UVB | Expo. aux UVB | Viabilité : 1 tissu |
| | | | | | | | Histologie : 1 tissu |
| | | | | | | | Mélanine : 3 tissus |

Les tissus sont placés à 36,5°C / 5% de CO₂ entre chaque application.

Un témoin positif est réalisé par l'application systémique d'alpha MSH sur dix tissus. Un témoin négatif est également réalisé avec de l'épiderme ne subissant aucune application (10 tissus également).

La moitié des tissus est exposée chaque jour à un rayonnement UV-B (50mJ/cm²) tandis que l'autre moitié ne subit aucun rayonnement.

Vingt-quatre heures après la dernière application, les tissus sont rincés. Un tissu par condition est alors utilisé pour l'étude histologique, un autre tissu est utilisé pour le test de viabilité cellulaire et les trois derniers tissus sont utilisés pour le dosage de la mélanine.

### b. Protocole du test de viabilité cellulaire :

Après 24 heures d'incubation, les tissus sont rincés trois fois avec 500 µl de PBS (Phosphate Buffer Saline) puis incubées à 36,5°C dans 300 µl de MTT (3-[4,5-dimethyl-thiazol-2-yl]-2,5-diphenyl tetrazolium bromide) pendant 3 heures.

Les inserts sont ensuite incubés à température ambiante dans de l'isopropanol au moins pendant une heure. Ensuite, une lecture de la densité optique (DO) à une longueur d'onde de 550 nm est réalisée.

### c. protocole de l'analyse histologique :

Après 24 heures d'incubation, un tissu de chaque condition est fixé afin de réaliser un marquage Fontana Masson.

### d. protocole de dosage de la mélanine :

Après 24 heures d'incubation, trois tissus de chaque condition sont utilisés afin de mesurer la mélanine :
- deux tissus pour le dosage de la mélanine après extraction soluble ; et
- un tissu pour déterminer l'index de Mélanine (suivant le Mexamêtre MX18).

### II. Résultats des tests :

### a. viabilité cellulaire :

Le tableau 4 ci-dessous reprend les résultats des tests de viabilité cellulaire pour les tissus non soumis aux rayonnements :

**Tableau 4 : résultats pour les tissus non soumis aux rayonnements :**

| | Témoin négatif (pas d'application) | | | Application de mélanine à 1% | | | Application d'alpha-MSH | | |
|---|---|---|---|---|---|---|---|---|---|
| DO 550nm | 1,004 | 1,058 | 1,024 | 1,101 | 1,117 | 1,092 | 1,064 | 1,192 | 1,148 |
| DO moyenne | 1,029 | | | 1,103 | | | 1,134 | | |
| Viabilité | 100% | | | 107,2% | | | 110,2% | | |

Le tableau 5 ci-dessous reprend les résultats des tests de viabilité cellulaire pour les tissus soumis aux rayonnements :

**Tableau 5 : résultats pour les tissus soumis aux rayonnements :**

| | Témoin négatif (pas d'application) | | | Application de mélanine à 1% | | | Application d'alpha-MSH | | |
|---|---|---|---|---|---|---|---|---|---|
| DO 550nm | 1,104 | 1,077 | 1,084 | 1,108 | 1,102 | 1,104 | 1,029 | 0,969 | 0,999 |
| DO moyenne | 1,088 | | | 1,104 | | | 0,999 | | |
| Viabilité | 100% | | | 101,5% | | | 91,8% | | |

Ainsi, il apparait que l'application répétée d'une solution de mélanine Olea à une concentration de 1% en poids du poids total de la solution ne réduit pas la viabilité cellulaire de l'épiderme testé.

En effet, la viabilité cellulaire est du même ordre que le témoin négatif.

De la même manière, les résultats du tableau 5 ci-dessous permettent de démontrer que l'exposition aux rayonnements UVB n'induit pas de modification de la viabilité des épidermes testés.

### b. Etude histologique :

Les figures la à 1f confirment les résultats obtenus au point a. ci-dessus concernant la viabilité cellulaire. Les tissus correspondent aux critères du Demandeur.
- la figure la correspond au témoin négatif, sans application ;
- la figure 1b correspond au tissu sur lequel de la mélanine à 1% est appliquée en topique ;
- la figure 1c correspond au tissu sur lequel de l'alpha-MSH est appliquée en systémique ;
- la figure 1d correspond au témoin négatif, sans application, mais soumis à un rayonnement UVB ;
- la figure le correspond au tissu sur lequel de la mélanine à 1% est appliquée en topique, et qui est soumis à un rayonnement UVB ;
- la figure 1f correspond au tissu sur lequel de l'alpha-MSH est appliquée en systémique, et qui est soumis à un rayonnement UVB.

En moyenne, tous les tissus contiennent, après 19 jours, cinq couches cellulaires avec une couche basale normale. On note cependant que la qualité des tissus ayant reçus plusieurs applications topiques de mélanine à 1% est plus faible. Ceci est probablement dû au fait que ces cellules sont couvertes par un liquide pendant la durée de l'expérience, ce qui entraine une moins bonne différentiation.

### c. Dosage de la mélanine :

Le dosage de la mélanine a été effectué en utilisant deux méthodes.

La mesure réalisée avec le mexamètre MX18 permet de connaitre la valeur de pigmentation des premières couches de l'épiderme, tandis que le dosage de la mélanine après extraction permet de connaitre la quantité totale de mélanine présente dans les tissus.

Le tableau 6 ci-dessous reprend les résultats des tests de dosage de la mélanine pour les tissus non soumis aux rayonnements (mexamètre MX18) :

**Tableau 6 :**

| | Témoin négatif (pas d'application) | Application de mélanine à 1% | Application d'alpha-MSH |
|---|---|---|---|
| Concentration mélanine (µg/ml) | 36 | 56 | 53,5 |
| Variation | / | +55,6% | +48,6% |

Le tableau 7 ci-dessous reprend les résultats des tests de dosage de la mélanine pour les tissus soumis aux rayonnements (mexamètre MX18) :

**Tableau 7 :**

| | Témoin négatif + UVB | Application de mélanine à 1% + UVB | Application d'alpha-MSH + UVB |
|---|---|---|---|
| Concentration mélanine (µg/ml) | 36 | 56 | 53,5 |
| Variation | / | +55,6% | +48,6% |

Grâce au mexamètre, Il a pu être montré que l'augmentation de la pigmentation, induite par l'application d'un extrait de mélanine d'Olea, est encore plus marquée lorsque l'on mesure la mélanine totale, qui augmente de 34% à 55% respectivement avec et sans rayonnement UVB.

Le tableau 8 ci-dessous reprend les résultats des tests de dosage de la mélanine totale pour les tissus non soumis aux rayonnements :

**Tableau 8 :**

| | Témoin négatif | Application de mélanine à 1% | Application d'alpha-MSH |
|---|---|---|---|
| Taux de mélanine total | 631,20 | 698,80 | 686,40 |
| Variation | / | +10,7% | +8,7% |

Enfin, le tableau 9 ci-dessous reprend les résultats des tests de dosage de la mélanine totale pour les tissus soumis aux rayonnements :

**Tableau 9 :**

| | Témoin négatif + UVB | Application de mélanine à 1% + UVB | Application d'alpha-MSH + UVB |
|---|---|---|---|
| Taux de mélanine total | 644,40 | 761,20 | 725,20 |
| Variation | / | +18,1% | +12,5% |

Le Demandeur a pu mettre en évidence que l'application de mélanine permet d'augmenter de 10% la pigmentation du tissu, ce qui est sensiblement identique au tissu témoin positif sur lequel de l'alpha-MSH est appliqué. Le Demandeur a par ailleurs pu observer que cette augmentation de la pigmentation passait à 18% lorsque le tissu est soumis à un rayonnement UVB.

Il ressorts également des résultats ci-dessus que la mélanine à 1% extraite d'Olea est bio-assimilée par les tissus.

## Revendications

1. Procédé de préparation d'un extrait d'olive à teneur en mélanine supérieure à 5% en poids du poids total de l'extrait comprenant les étapes suivantes de :
- mélange d'une matière première issue de l'olive choisie parmi des grignons d'olives noires et/ou des margines d'olives noires avec une solution aqueuse alcaline, ladite matière première a une concentration en lipides inférieure ou égale à 10% en poids du poids total de ladite matière ;
- macération du mélange obtenu ;
- filtration du macérât ;
- mélange d'un composé acide au filtrat obtenu ;
- filtration du mélange afin d'obtenir un gâteau humide ;
- séchage du gâteau humide ; et
- récupération d'un extrait solide comprenant une teneur en mélanine supérieure à 5% en poids du poids total de l'extrait.

2. Procédé selon la revendication 1 **caractérisé en ce que** la solution aqueuse alcaline est une solution aqueuse de soude (NaOH) ou de potasse (KOH).

3. Procédé selon l'une des revendications précédentes, **caractérisé en ce que** le pH du milieu alcalin est compris entre 8 et 12.

4. Procédé selon l'une des revendications précédentes, **caractérisé en ce que** le composé acide est choisi parmi l'acide citrique (acide 2-hydroxypropane-1,2,3-tricarboxylique), l'acide acétique (acide éthanoïque), l'acide sulfurique (sulfate d'hydrogène) ou l'acide chlorhydrique (HCl).

5. Extrait d'olive à teneur en mélanine supérieure à 5% en poids du poids total de l'extrait susceptible d'être obtenu par le procédé selon l'une des revendications 1 à 4.

## Patentansprüche

1. Verfahren zur Herstellung eines Olivenextrakts mit einem Melaningehalt von mehr als 5 Gew.-% des Gesamtgewichts des Extrakts, umfassend die folgenden Schritte:
- Mischen eines Rohstoffs, hervorgegangen aus Olive, ausgewählt aus Trestern schwarzer Oliven und/oder Fruchtwassern schwarzer Oliven, mit einer alkalischen wässrigen Lösung, wobei der Rohstoff eine Lipidkonzentration von unter oder gleich 10 Gew.-% des Gesamtgewichts des Rohstoffs hat;
- Mazerieren des erhaltenen Gemischs;
- Filtration des Mazerats;
- Mischen einer sauren Verbindung mit dem erhaltenen Filtrat;
- Filtration des Gemischs, um einen feuchten Kuchen zu erhalten;
- Trocknen des feuchten Kuchens; und
- Rückgewinnen eines festen Extrakts, umfassend einen Melaningehalt von mehr als 5 Gew.-% des Gesamtgewichts des Extrakts.

2. Verfahren nach Anspruch 1, **dadurch gekennzeichnet, dass** die alkalische wässrige Lösung eine wässrige Natron-(NaOH) oder Kalilösung (KOH) ist.

3. Verfahren nach einem der vorangehenden Ansprüche, **dadurch gekennzeichnet, dass** der pH des alkalischen Milieus zwischen 8 und 12 liegt.

4. Verfahren nach einem der vorangehenden Ansprüche, **dadurch gekennzeichnet, dass** die saure Verbindung aus der Citronensäure (2-Hydroxypropan-1,2,3-tricarbonsäure), der Essigsäure (Ethansäure), der Schwefelsäure (Hydrogensulfat) oder der Chlorwasserstoffsäure (HCl) ausgewählt ist.

5. Olivenextrakt mit einem Melaningehalt von mehr als 5 Gew.-% des Gesamtgewichts des Extrakts, der durch das Verfahren nach einem der Ansprüche 1 bis 4 gewinnbar ist.

## Claims

1. Method for preparing an olive extract with a melanin content greater than 5 wt% of the total weight of the extract, comprising the steps of:
- mixing a raw material derived from olives chosen from the group consisting of olive cakes of black olives and/or black liquors of black olives with an alkaline aqueous solution, said raw material having a lipid concentration of less than or equal to 10 wt% of the total weight of said material;
- macerating the mixture obtained;
- filtering the macerate;
- mixing an acidic compound with the filtrate obtained;
- filtering the mixture to obtain a wet cake;
- drying the wet cake; and
- recovering a solid extract with a melanin content greater than 5 wt% of the total weight of the extract.

2. Method according to claim 1, **characterised in that** the alkaline aqueous solution is an aqueous solution of sodium hydroxide (NaOH) or of potassium hydroxide (KOH).

3. Method according to one of the preceding claims, **characterised in that** the pH of the alkaline medium lies in the range 8 to 12.

4. Method according to one of the preceding claims, **characterised in that** the acidic compound is chosen from the group consisting of citric acid (2-hydroxypropane-1,2,3-tricarboxylic acid), acetic acid (ethanoic acid), sulphuric acid (hydrogen sulphate) or hydrochloric acid (HCl).

5. Olive extract with a melanin content greater than 5 wt% of the total weight of the extract capable of being obtained by the method according to one of claims 1 to 4.
